(19) Europäisches Patentamt
European Patent Office
Office européen des brevets

(11) Numéro de publication : **0 165 226**
**B1**

(12) # FASCICULE DE BREVET EUROPÉEN

(45) Date de publication du fascicule du brevet : **15.03.89**

(51) Int. Cl.⁴ : **C 07 C125/065**

(21) Numéro de dépôt : **85870080.0**

(22) Date de dépôt : **04.06.85**

(54) **Nouveaux dérivés d'acide amino-4 butanoique, leur procédé de préparation ainsi que leur utilisation.**

(30) Priorité : **06.06.84 FR 8408868**

(43) Date de publication de la demande : **18.12.85 Bulletin 85/51**

(45) Mention de la délivrance du brevet : **15.03.89 Bulletin 89/11**

(84) Etats contractants désignés : **AT BE CH DE FR GB IT LI LU NL SE**

(56) Documents cités :
**JUSTUS LIEBIGS: "ANNALEN DER CHEMIE", fascicule 12, 1975, pages 2245-2250, Weinheim, DE; R. STEULMANN et al.: "Synthesen der (3S,4S)-4-amino-3-hydroxy-6-methylheptansäure und einiger Derivate"**

(73) Titulaire : **SANOFI**
**40, Avenue George V**
**F-75008 Paris (FR)**

(72) Inventeur : **Descamps, Marcel**
**Place des Carmes 8/301**
**B-1300 Wavre (BE)**
Inventeur : **Verstraeten, Walter**
**Zwijvegemstraat 10**
**B-2960 Mechelen (BE)**

(74) Mandataire : **Bressand, Georges et al**
**c/o CABINET LAVOIX 2 Place d'Estienne d'Orves**
**F-75441 Paris Cedex 09 (FR)**

EP 0 165 226 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention se rapporte, d'une manière générale, à de nouveaux dérivés d'acide amino-4 butanoïque de formule générale :

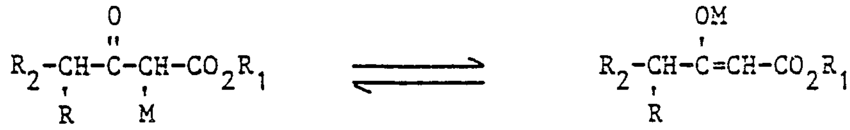

(I) (I')

dans laquelle :

M représente un atome de métal alcalin, par exemple lithium, sodium ou potassium,

R représente un groupement amino protégé,

$R_1$ représente un groupement labile,

$R_2$ représente hydrogène, un radical alkyle, linéaire ou ramifié, ayant de 1 à 6 atomes de carbone tels que méthyle, éthyle, n-propyle, isopropyle, n-butyle, isobutyle, tertiobutule, méthyl-1 propyle, n-pentyle ou n-hexyle, un radical méthyloxyalkyle inférieur tel que méthyloxyéthyle, un radical méthylthioalkyle inférieur tel que méthylthiométhyle, un radical (alkyle inférieur) aminoalkyle inférieur tel que méthylamino-, éthylamino-, isopropylamino- ou tertiobutylamino-éthyle, un radical (dialkyle inférieur) aminoalkyle inférieur tel que diméthylamino-, diéthylamino-, di-n-propylamino- ou di-n-butylamino-éthyle, un radical hydroxyalkyle inférieur tel qu'hydroxyméthyle ou hydroxy-1 éthyle ou $R_2$ représente l'un des radicaux de formules générales :

$$Cy—A—, \quad Cy—O—A'—, \quad R—A'— \text{ ou } R_3S—A'—$$

dans lesquelles :

Cy représente un radical hydrocarboné aromatique ou alicyclique ou un radical hétérocycle contenant un atome d'oxygène ou de soufre ou un ou deux atomes d'azote, Cy étant éventuellement mono-, di- ou tri-substitué par des radicaux hydroxy, alkyle inférieur, alkoxy inférieur, trifluorométhyle, nitro ou halogéno,

A représente une liaison simple ou un radical alkylène, linéaire ou ramifié, ayant de 1 à 5 atomes de carbone,

A' représente un radical alkylène, linéaire ou ramifié, ayant de 1 à 5 atomes de carbone,

$R_3$ représente un groupement S-protecteur.

Dans le présent contexte, les termes repris ci-dessous comportent la signification suivante :

« groupement amino protégé » désigne un groupement phtalimido, un groupement amino protégé par un proton ou encore un groupement amino protégé par des groupements protecteurs facilement éliminables pour groupements amino, tels que, à titre d'exemple, un groupement alkylecarbonyle tels que formyle, acétyle ou propionyle, un groupement alkoxycarbonyle tel que tertiobutoxycarbonyle, un groupement alkoxyalkylcarbonyle tel que méthoxyacétyle ou méthoxypropionyle, un groupement alkoxycarbonyle substitué tel que trichloro-2,2,2 éthoxycarbonyle, un groupement aralkyloxycarbonyle tel que benzyloxycarbonyle, un groupement aralkyloxycarbonyle substitué tel que p-nitrobenzyloxycarbonyle, un groupement trityle ou méthoxytrityle ou un groupement arylsulfonyle tel que p-toluènesulfonyle. Le groupement tertiobutoxycarbonyle (BOC) constitue un groupement préféré ;

« alkyle inférieur » désigne les restes d'hydrocarbures aliphatiques saturés contenant jusqu'à 4 atomes de carbone tels que méthyle, éthyle, n-propyle, isopropyle, n-butyle, isobutyle ou tertiobutyle ; « alkoxy inférieur » désigne le groupement hydroxy substitué par un radical alkyle inférieur tel que défini ci-dessus ;

« groupement labile » désigne un groupement facilement éliminable tel qu'un groupement alkyle inférieur défini ci-dessus ou un groupement aralkyle substitué ou non tel que benzyle ou xylyle ;

« radical hydrocarboné aromatique ou alicyclique » signifie un radical phényle, naphtyle, cycloalkyle de 3 à 7 atomes de carbone tels que cyclopropyle, cyclobutyle, cyclopentyle, cyclohexyle ou cycloheptyle ;

« radical hétérocyclique » signifie un radical furyle, benzofuryle, thiényle, benzothiényle, pyrrolyle, pyridyle, pipéridyle, morpholyle, pipérazinyle, N-(alkyle inférieur)-pipérazinyle, indolyle ou 1H-imidazolyle ;

« groupement S-protecteur » désigne un groupement facilement éliminable pour groupements mercapto tels que par exemple, un groupement aralkyle tel que benzyle, un groupement aralkyle substitué tel que p-nitrobenzyle, un groupement alkoxycarbonyle substitué tel que trichloro-2,2,2 éthoxycarbonyle, un groupement aralkylthioalkyle tel que benzylthiométhyle, un groupement aralkyloxycarbonyle substitué tel que p-méthoxybenzyloxycarbonyle, un groupement picolyle tel que γ-picolyle, un groupement alkylcarbamoyle tel qu'éthylcarbamoyle, un groupement alkoxyalkylcarbamoyle tel que

méthoxyméthylcarbamoyle, un groupement alkanecarboxamidoalkyle tel que méthane-carboxamidométhyle, un groupement diarylalkyle tel que diphénylméthyle, un groupement hétérocyclique oxygéné tel que dihydropyrannyle ou tétrahydrofurannyle ou un groupement trityle.

Ainsi, en tenant compte des valeurs données ci-dessus :

— le radical Cy—A— peut représenter notamment un radical phényle, hydroxyphényle tel qu'hydroxy-4 phényle, un radical méthylphényle, mono-fluoro-, mono-chloro- ou mono-bromo-phényle, di-fluoro-, di-chloro- ou di-bromo-phényle, mono-méthoxy, di-méthoxy- ou tri-méthoxy-phényle, trifluorométhyl-phényle, nitrophényle, benzyle, phénéthyle, cyclohexyle, cyclohexylméthyle, picolyle, pipéridinométhyle, indolylméthyle tel qu'indolyl-3 méthyle ou 1H-imidazolylméthyle tel qu'1H-imidazolyl-4 méthyle,

— le radical Cy—O—A'— peut représenter notamment un radical phénoxyéthyle ou phénoxy-n-propyle,

— le radical R—A'— peut représenter notamment un radical amino-4 butyle N-protégé,

— le radical $R_3S$—A'— peut représenter notamment un radical mercaptométhyle S-protégé.

Des composés représentatifs de la présente invention sont ceux de formule I-I' dans laquelle R a la signification donnée, en particulier —NHBOC, $R_1$ a la signification donnée en particulier méthyle ou éthyle, M a la signification donnée en particulier sodium et $R_2$ représente un radical méthyle, éthyle, n-propyle, isopropyle, n-butyle, isobutyle, phényle, p-hydroxyphényle, benzyle, cyclohexyle, cyclohexylméthyle, indolyl-3 méthyle ou 1H-imidazolyl-4 méthyle.

Les composés de formule I-I' dans laquelle R, $R_1$ et M ont la signification donnée et $R_2$ représente isobutyle constituent des composés préférés de l'invention, en particulier les (4S)-oxo-3 N—BOC-amino-4 méthyl-6 heptanoate de méthyle, (4S)-oxo-3 N—BOC-amino-4 méthyl-6 heptanoate d'éthyle, (4S)-oxo-3 N—BOC-amino-4 méthyl-6 heptanoate de n-propyle, (4S)-oxo-3 N—BOC-amino-4 méthyl-6 heptanoate d'isopropyle, (4S)-oxo-3 N—BOC-amino-4 méthyl-6 heptanoate de n-butyle, (4S)-oxo-3 N—BOC-amino-4 méthyl-6 heptanoate d'isobutyle, (4S)-oxo-3 N—BOC-amino-4 méthyl-6 heptonoate de tertiobutyle et (4S)-oxo-3 N—BOC-amino-4 méthyl-6 heptanoate de benzyle.

Les composés de l'invention se sont révélés particulièrement utiles comme produits intermédiaires notamment pour la synthèse finale des dérivés d'acide (3S,4S)-hydroxy-3 amino-4 butanoïque de formule générale :

$$R_2-\underset{R}{CH}-\overset{OH}{CH}-CH_2-CO_2H \qquad (a)$$

dans laquelle R et $R_2$ ont la signification donnée ci-dessus, en particulier pour la synthèse finale de l'acide (3S,4S)-hydroxy-3 amino-4 méthyl-6 heptanoïque ou statine.

Un premier objet de l'invention se rapporte, en conséquence, aux dérivés d'acide amino-4 butanoïque de formule I-I' en tant que produits industriels nouveaux utiles notamment comme intermédiaires par exemple pour la synthèse finale des acides de formule (a) ci-dessus en particulier pour la synthèse finale de la statine.

Pour des raisons de commodité, on a adopté, par la suite, une nomenclature basée sur la forme β-cétonique des composés de l'invention représentée par la formule I.

On connaît des peptides dérivés d'analogues d'acide amino-4 butanoïque et notamment dérivés de la statine utiles pour leurs potentialités antihypertensives par inhibition de l'enzyme de conversion de la rénine en angiotensine. De tels composés, analogues de pepstatine, ont été décrits par exemple dans le brevet U.S. No. 4.485.099 ou la demande de brevet européen No. 104.954.

La synthèse de peptides de ce type nécessite une voie d'accès aisée vers les acides de formule (a) et en particulier vers la statine par l'intermédiaire de composés dont les fonctions réactives sont bloquées par des groupements labiles.

Dans le cas particulier de la statine, de tels composés peuvent être, par exemple des esters alkyliques d'acide (3S,4S)-hydroxy-3 N—BOC-amino-4 méthyl-6 heptanoïque tels que l'ester méthylique [Bull. Soc. Chim. France, No. 7-8, pp. 230-232 (1983)] ou l'ester éthylique (Liebigs Ann. Chem., 1975, p. 2245).

On a cité dans cette dernière référence, un procédé pour la préparation d'un mélange de deux diastéréoisomères d'hydroxy-3 N—BOC-amino-4 méthyl-6 heptanoate d'éthyle en l'occurrence les isomères (3S,4S) et (3R,4S) en faisant intervenir, comme l'un des produits intermédiaires, le (4S)-oxo-3 N—BOC-amino-4 méthyl-6 heptanoate d'éthyle.

Toutefois, la séparation du mélange d'isomères obtenu n'a pas été réalisée. Suivant le procédé décrit le (4S)-oxo-3 N—BOC-amino-4 méthyl-6 heptanoate d'éthyle est préparé à partir de l'énolate magnésien du malonate acide d'éthyle qui est condensé dans un éther sur le N—BOC—L-leucine imidazolide.

Le dérivé d'heptanoate d'éthyle ainsi obtenu se présente sous forme huileuse cristallisant uniquement après plusieurs semaines. En outre, le rendement après purification par recristallisation fractionnée est faible, de l'ordre de 36,6 %.

L'hydrogénation de cet ester éthylique est ensuite réalisée, fournissant un mélange de (3S,4S ; 3R,4S)-hydroxy-3 N—BOC-amino-4 méthyl-6 heptanoate d'éthyle avec un rendement de 32,5 % calculé à partir de la N—BOC—L-leucine.

De même, on a décrit dans Eur. J. Med. Chem., 1978, 13, No. 5, pp. 429-434, un procédé analogue à celui cité précédemment selon lequel on hydrogène le (4R) ou (4S)-oxo-3 N—BOC-amino-4 pentanoate d'éthyle pour obtenir un mélange, apparemment non purifié ni séparé, de (3S,4S ; 3R,4S)-hydroxy-3 N—BOC-amino-4 pentanoate d'éthyle avec un rendement ne dépassant pas les 50 % calculé à partir de la (+)—BOC—D-alanine ou de la (—)—BOC—L-alanine.

On a trouvé, dans le cadre de la présente invention, que les sels de métaux alcalins de formule I-I' constituent des produits intermédiaires particulièrement intéressants pour la préparation des mélanges d'esters de dérivés d'acide (3S,4S ; 3R,4S) d'hydroxy-3 amino-4 butanoïque N-protégés correspondants et en particulier pour la préparation des mélanges (3S,4S ; 3R,4S) d'hydroxy-3 amino-4 méthyl-6 heptanoates d'alkyle inférieur N-protégés correspondants puisqu'ils permettent d'obtenir ces composés avec un haut degré de pureté et avec des rendements de loin supérieurs à ceux obtenus selon la technique antérieure.

Ces mélanges peuvent être séparés avec facilité en leurs diastéréoisomères pour fournir l'isomère (3S,4S) désiré sous forme particulièrement pure. Par exemple, la préparation de mélanges d'isomères d'hydroxy-3 amino-4 méthyl-6 heptanoates d'alkyle inférieur, peut être réalisée au départ de composés de formule I-I' ci-dessus, où R représente BOC—NH— et $R_2$ représente isobutyle et ce, avec un rendement d'au moins 55 % calculé à partir de la N—BOC—L-leucine.

Parmi les isomères (3S,4S) des hydroxy-3 N—BOC-amino-4 méthyl-6 heptanoates d'alkyle inférieur dont question ci-dessus, l'isomère (3S,4S) de l'hydroxy-3 N—BOC-amino-4 méthyl-6 heptanoate de méthyle se présente sous forme cristalline particulièrement avantageuse.

En conséquence, les composés de formule I-I' capables de donner accès à cet isomère constitueront des composés préférés selon l'invention, c'est-à-dire les composés de formule I-I' dans laquelle R représente le radical BOC—NH—, $R_1$ représente le radical méthyle, $R_2$ représente le radical isobutyle et M représente le sodium.

Les dérivés d'acide amino-4 butanoïque de l'invention peuvent être obtenus à partir d'un imidazolide N-protégé de formule générale :

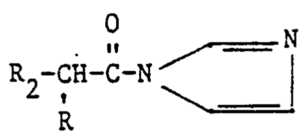

(II)

dans laquelle R et $R_2$ ont la même signification que précédemment.

On fait tout d'abord réagir cet imidazolide de formule II avec un énolate magnésien d'un mono ester d'acide malonique de formule générale :

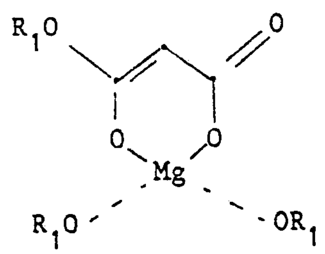

(III)

dans laquelle $R_1$ a la même signification que précédemment, la réaction ayant lieu à température ambiante et dans un éther tel que le tétrahydrofuranne éventuellement en présence d'un solvant aprotique tel que le diméthylsulfoxyde ou le N,N-diméthylformamide pour donner un complexe que l'on hydrolyse en présence d'un acide fort par exemple l'acide chlorhydrique pour ainsi fournir les esters de dérivés d'acide (4S)-oxo-3 amino-4 butanoïque N-protégés de formule générale :

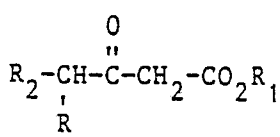

(IV)

dans laquelle R, $R_1$ et $R_2$ ont la même signification que précédemment.

Un tel mélange éther/solvant aprotique est particulièrement indiqué pour la préparation des composés de formule IV dans laquelle $R_1$ représente méthyle et $R_2$ représente isobutyle notamment pour la préparation du (4S)-oxo-3 amino-4 méthyl-6 heptanoate de méthyle.

Dans ce cas, on utilise de préférence un mélange tétrahydrofuranne/diméthylsulfoxyde qui permet

l'obtention de rendements souvent supérieurs à 60 % en produit désiré sous forme pure alors que dans le tétrahydrofuranne seul, les rendements ne sont que de 25 à 30 %.

On forme alors le sel de métal alcalin de formule I-I' par réaction des dérivés butanoates de formule IV en milieu aqueux, avec un hydroxyde de métal alcalin à température inférieure à 20 °C et on sépare le sel formé du milieu réactionnel.

Les composés de formule II peuvent être préparés à partir d'un acide aminé N-protégé de formule générale :

$$R_2-\underset{\displaystyle R}{\underset{|}{CH}}-\overset{\displaystyle O}{\overset{\|}{C}}-OH \qquad (V)$$

dans laquelle R et $R_2$ ont la même signification que précédemment et de N,N-thionyldiimidazole suivant une méthode analogue à celle décrite dans Bull. Soc. Chim. France 1964, pp. 945-951.

Les composés de formule V sont soit des produits connus soit des produits pouvant être préparés en protégeant de manière classique le groupement amino et éventuellement mercapto, des acides aminés L correspondants.

Ces acides aminés sont des composés naturels connus ou des analogues de ces composés.

De tels acides aminés ainsi que leur préparation ont été décrits dans les brevets belges Nos. 883.640 ou 845.187.

Les sels de métaux alcalins de l'invention, ainsi obtenus peuvent être utilisés notamment pour la régénération des esters de dérivés d'acide (4S)-oxo-3 amino-4 butanoïque N-protégés correspondants et ce, par acidification au moyen d'un acide fort, par exemple l'acide chlorhydrique.

Dans cette application, l'isolation intermédiaire des sels de métaux alcalins de l'invention constitue une méthode de choix pour la purification des esters de dérivés d'acide (4S)-oxo-3 amino-4 butanoïque N-protégés de formule IV obtenus à l'état brut lors de la mise en œuvre du procédé de préparation décrit précédemment.

En conséquence, un autre objet de l'invention concerne l'application des dérivés d'acide butanoïque de formule I-I' à la purification des esters de dérivés d'acide (4S)-oxo-3 amino-4 butanoïque N-protégés correspondants, obtenus à l'état brut, consistant à former, au moyen d'un hydroxyde de métal alcalin, le sel de métal alcalin des esters de dérivés d'acide (4S)-oxo-3 amino-4 butanoïque N-protégés en question, à isoler le sel formé puis à régénérer les esters de dérivés d'acide (4S)-oxo-3 amino-4 butanoïque N-protégés par acidification au moyen d'un acide fort.

Cette purification par passage au sel de métal alcalin correspondant permet d'obtenir les esters de dérivés d'acide (4S)-oxo-3 amino-4 butanoïque N-protégés avec des rendements importants. Dans le cas des (4S)-oxo-3 amino-4 méthyl-6 heptanoates d'alkyle inférieur, ceux-ci ont été trouvés supérieurs à 60 % calculés au départ de la L-leucine N-protegée. Aussi, la purification des composés de formule IV par passage transitoire à leurs sels de métaux alcalins isolés constitue-t-elle un avantage indéniable sur la méthode de purification par cristallisation fractionnée selon la technique antérieure. Les esters de dérivés d'acide (4S)-oxo-3 amino-4 butanoïque N-protégés ainsi purifiés pourront être réduits ultérieurement, par exemple par l'hydrogène catalytique pour donner des esters de dérivés d'acide (3S,4S ; 3R,4S)-hydroxy-3 amino-4 butanoïque N-protégés sous forme de mélanges de diastéréoisomères pratiquement exempts de produits secondaires.

Il est possible, à ce stade, d'effectuer d'une manière presque quantitative la séparation des deux diastéréoisomères par exemple par chromatographie. On a constaté, en outre, que la préparation des esters de dérivés d'acide (3S,4S ; 3R,4S)-hydroxy-3 amino-3 amino-4 butanoïque N-protégés peut être effectuée directement à partir des sels de formule I-I' par réduction de ces composés par exemple par l'hydrogène catalytique.

De cette manière, on évite une étape de procédé à savoir la régénération des esters de dérivés d'acide (4S)-oxo-3 amino-4 butanoïque N-protégés à partir de leurs sels de métaux alcalins.

Ainsi, un autre objet de l'invention concerne l'application des dérivés d'acide butanoïque de formule I-I' à la préparation des diastéréoisomères (3S,4S) et (3R,4S) des esters de dérivés d'acide hydroxy-3 amino-4 butanoïqueN-protégés correspondants consistant à réduire les composés de formule I-I' par exemple par l'hydrogène catalytique, pour obtenir les esters de dérivés d'acide (3S,4S ; 3R,4S)-hydroxy-3 amino-4 butanoïque N-protégés sous forme de mélanges de diastéréoisomères puis à séparer les diastéréoisomères en question par exemple par chromatographie.

Les rendements enregistrés avec cette variante sont analogues à ceux obtenus lors de la réduction des esters de dérivés d'acide (4S)-oxo-3 amino-4 butanoïque N-protégés purifiés par passage intermédiaire au sel de métal alcalin, c'est-à-dire supérieurs à 90 % dans le cas des (4S)-oxo-3 amino-4 méthyl-6 heptanoates d'alkyle inférieur N-protégés. En conséquence, les avantages apportés par les sels de métaux alcalins de l'invention constituent des améliorations indéniables à la technique connue pour la préparation d'esters de dérivés d'acide hydroxy-3 amino-4 butanoïque N-protégés dans l'optique d'une extrapolation sur le plan industriel.

Les Exemples non limitatifs suivants illustrent l'invention :

Préparations

A) N,N'-thionyldiimidazole

Dans un ballon d'un litre muni d'un agitateur et d'une ampoule à décanter fermée par une garde desséchante, on place 13,6 g (0,2 mole) d'imidazole dissous dans 150 ml de tétrahydrofuranne.

On ajoute alors, sous agitation, une solution de 6 g de chlorure de thionyle dans 50 ml de tétrahydrofuranne.

La précipitation du chlorhydrate d'imidazole commence immédiatement.

Après 20 minutes d'agitation, on filtre et on lave le précipité avec 50 ml de tétrahydrofuranne. On obtient ainsi une solution limpide de N,N'-thionyldiimidazole utilisée telle quelle pour l'opération suivante.

B) Imidazolide de la N—BOC—L-leucine

Dans un ballon d'un litre muni d'un agitateur et d'une ampoule à décanter, on place la solution obtenue précédemment puis on ajoute, goutte à goutte, une solution de 11,5 g (0,05 mole) de N—BOC—L-leucine dans 20 ml de tétrahydrofuranne.

On poursuit l'agitation durant 20 min. en évacuant par succion sous vide (environ 20 mm Hg) l'anhydride sulfureux formé. On obtient ainsi une solution légèrement trouble d'imidazolide de la N-BOC—L-leucine utilisé brut.

C) Malonate acide de méthyle

Dans un ballon de 4 l, muni d'un agitateur et d'une ampoule à décanter, on place 660 g (5 moles) de malonate de diméthyle. On ajoute alors en 8 h environ, une solution à 20 °C de 281 g d'hydroxyde de potassium dans 2 l de méthanol.

Après 15 à 16 heures d'agitation à la température ambiante, on filtre le précipité de sel potassique du malonate acide de méthyle et on le lave soigneusement à l'éther éthylique.

On reprend le précipité par 750 ml d'eau et on acidifie avec de l'acide chlorhydrique dilué jusqu'à pH = 2 à 3 tout en refroidissant par un mélange glace/méthanol. On extrait 3 fois avec de l'éther éthylique, sèche la phase éthérée et évapore à sec.

On isole ainsi 156 g de malonate acide de méthyle brut, ce qui représente un rendement de 26 %.

Après une distillation sous $5 \cdot 10^{-2}$ Torr à 80-85 °C, on isole 135 g de produit pur ce qui représente un rendement de 23 %.

$n_D^{23} = 1{,}4300$

R.M.N. : conforme.

Dosage protométrique : 97,17 %.

De la même manière mais au départ de malonate de diéthyle, on prépare le malonate acide d'éthyle.

D) Enolate magnésien du malonate acide de méthyle

Dans un ballon de 2 l, muni d'un agitateur, d'un réfrigérant et d'une ampoule à décanter, on introduit successivement 4,8 g (0,2 mole) de magnésium, 0,2 ml de tétrachlorure de carbone et 10 ml de méthanol.

A ce mélange, on ajoute, sous agitation, 10 ml d'une solution de 23,5 g (0,2 mole) de malonate acide de méthyle dans 50 ml de méthanol.

La réaction démarre spontanément. Lorsqu'elle se calme, on ajoute le reste de la solution de malonate de façon à tenir un léger reflux. Après introduction, on chauffe au bain-marie durant 8 heures, on ajoute 200 ml de tétrahydrofuranne et on poursuit le chauffage au bain-marie durant 12 heures. On distille alors les solvants, d'abord à pression atmosphérique ensuite sous 20 mm Hg environ. Arrivé à sec, on ajoute 100 ml de benzène et on distille à pression atmosphérique puis sous vide. Finalement, on ajoute 100 ml de tétrahydrofuranne.

On obtient ainsi une suspension d'énolate magnésien du malonate acide de méthyle.

De la même manière, mais au départ de malonate acide d'éthyle, on prépare l'énolate magnésien du malonate acide d'éthyle.

Exemple 1

Préparation du sel sodique de (4S)-oxo-3 N—BOC-amino-4 méthyl-6 heptanoate de méthyle

On prépare l'imidazolide de la N—BOC—L-leucine à partir de 13,6 g d'imidazole et de 11,5 g de N—BOC—L-leucine suivant la méthode décrite au paragraphe B précédent. De même, on prépare l'énolate magnésien du malonate acide de méthyle à partir de 24,7 g de malonate acide de méthyle selon

la méthode décrite au paragraphe D précédent.

On ajoute la solution d'imidazolide à la suspension d'énolate magnésien dans le tétrahydrofuranne puis on ajoute 130 ml de diméthylsulfoxyde. Le mélange se clarifie et tout se dissout.

Après 4 heures d'agitation à température ambiante, on acidifie au moyen d'acide chlorhydrique 1M jusqu'à pH neutre. On agite 30 minutes à température ambiante afin de parfaire l'hydrolyse.

On décante et on extrait 3 fois à l'éther éthylique. On lave la phase éthérée successivement avec de l'eau, de l'eau bicarbonatée puis avec de l'eau.

Après séchage sur sulfate de sodium, on évapore à sec et on obtient 12,2 de (4S)-oxo-3 N—BOC-amino-4 méthyl-6 heptanoate de méthyle brut (rendement : 86 %).

On reprend le dérivé d'heptanoate de méthyle brut dans 60 ml d'hexane et 20 ml d'eau. On agite le mélange hétérogène et à température inférieure à 20 °C, on ajoute 10 ml d'une solution d'hydroxyde de sodium à 30 %. La précipitation du sel sodique commence après environ 5 minutes. Afin de compléter la précipitation, on continue l'agitation durant 15 minutes.

On filtre le précipité et on le lave avec de l'eau glacée et de l'éther éthylique. Après séchage, on obtient 9,5 g de sel sodique de (4S)-oxo-3 N—BOC-amino-4 méthyl-6 heptanoate de méthyle, légèrement soluble dans l'éther éthylique.

Rendement : 62 % calculé au départ de la N—BOC—L-leucine

P.F. : 187 °C (décomposition)

Analyse

$C_{14}H_{24}NO_5Na$

Calculé : C 54,36 H 7,82 N 4,53 %

Trouvé : C 54,22 H 7,90 N 4,70 %

Dosage acidobasique : 100,6 %

$\alpha_D^{25}$ = — 11,79° (C = 1, méthanol)

R.M.N. et C.C.M. : conformes

De manière semblable à celle décrite précédemment, on a préparé les composés suivants :

Sel sodique de (4S)-oxo-3 N—BOC-amino-4 phényl-5 pentanoate de méthyle

P.F. : 195-197 °C.

Sel sodique de (4S)-oxo-3 N—BOC-amino-4 cyclohexyl-5 pentanoate de méthyle

P.F. : 120-122 °C.

Sel sodique de (4S)-oxo-3 N—BOC-amino-4 phényl-4 butyrate de méthyle

P.F. : 182-184 °C.

Sel sodique de (4S)-oxo-3 N—BOC-amino-4 méthyl-5 hexanoate de méthyle soluble dans l'eau.

Sel sodique de (4S)-oxo-3 N—BOC-amino-4 octanoate de méthyle

P.F. : 200-202 °C.

Sel sodique de (4S)-oxo-3 N—BOC-amino-4 pentanoate de méthyle

$\alpha_D^{21}$ = — 10,0° (C = 1, méthanol)

Sel sodique de (4S)-oxo-3 N—BOC-amino-4 hexanoate de méthyle

Sel sodique de (4S)-oxo-3 N—BOC-amino-4 cyclohexyl-4 butyrate de méthyle

Sel sodique de (4S)-oxo-3 N—BOC-amino-4 (hydroxy-4 phényl)-5 pentanoate de méthyle

Sel sodique de (4S)-oxo-3 N—BOC-amino-4 (1H-imidazolyl-4)-5 pentanoate de méthyle

## Exemple 2

Préparation du sel sodique de (4S)-oxo-3 N—BOC-amino-4 méthyl-6 heptanoate d'éthyle

Dans un ballon de 2 l, on agite un mélange de N—BOC-L-leucine imidazolide (préparé au départ de 77 g d'imidazole et de 65,5 g (0,28 mole) de N—BOC—L-leucine anhydre suivant la méthode décrite au paragraphe B précédent) et d'énolate magnésien du malonate acide d'éthyle (préparé au départ de 151 g de malonate acide d'éthyle suivant la méthode décrite au paragraphe D précédent).

Après cette opération, on acidifie la suspension par de l'acide chlorhydrique 1M jusqu'à pH neutre. On agite durant 30 minutes à température ambiante afin de parfaire l'hydrolyse. On décante et extrait 3 fois à l'éther éthylique. On lave la phase éthérée successivement avec de l'eau, de l'eau bicarbonatée puis avec de l'eau. Après séchage sur sulfate de sodium, on évapore à sec et on obtient le (4S)-oxo-3 N—BOC-amino-4 méthyl-6 heptanoate d'éthyle sous forme brute.

On reprend le dérivé d'heptanoate d'éthyle brut avec 60 ml d'éther éthylique et 60 ml d'eau. On agite le mélange hétérogène et à température inférieure à 20 °C, on ajoute 30 ml d'une solution d'hydroxyde de sodium à 30 % et on poursuit l'agitation durant 15 à 20 minutes. On filtre le précipité et on le lave avec de l'eau glacée et de l'éther éthylique.

Après séchage, on obtient 56,5 g de sel sodique de (4S)-oxo-3 N—BOC-amino-4 méthyl-6 heptanoate d'éthyle.

Rendement : 62,2 % calculé au départ de la N—BOC—L-leucine.

P.F. : 180 °C (décomposition)

Les Exemples suivants illustrent l'utilisation des composés de l'invention

Exemple I

Préparation du (4S)-oxo-3 N—BOC-amino-4 méthyl-6 heptanoate de méthyle

On prépare l'imidazolide de la N—BOC—L-leucine à partir de 136 g d'imidazole et 115 g de N—BOC—L-leucine suivant la méthode décrite au paragraphe B précédent. De même on prépare l'énolate magnésien du malonate acide de méthyle à partir de 236 g de malonate acide de méthyle selon la méthode décrite au paragraphe D précédent.

On ajoute la solution d'imidazolide à la suspension d'énolate magnésien dans le tétrahydrofuranne puis on ajoute 1,3 l de diméthylsulfoxyde.

Le mélange se clarifie et tout se dissout.

Après 4 heures d'agitation à température ambiante, on acidifie au moyen d'acide chlorhydrique 1M jusqu'à pH neutre. On agite durant 30 minutes à température ambiante afin de parfaire l'hydrolyse. On décante et on extrait 3 fois à l'éther éthylique. On lave la phase éthérée successivement avec de l'eau, de l'eau bicarbonatée puis avec de l'eau. Après séchage sur sulfate de sodium, on évapore à sec et on obtient 129 g de (4S)-oxo-3 N—BOC-amino-4 méthyl-6 heptanoate de méthyle brut (rendement : 89 %).

On reprend le dérivé d'heptanoate de méthyle brut dans 200 ml d'éther isopropylique, 200 ml d'hexane et 100 ml d'eau.

A température inférieure à 20 °C, on ajoute sous agitation, 50 ml d'hydroxyde de sodium à 30 %. Après 15 min. d'agitation, on filtre le précipité de sel sodique de (4S)-oxo-3 N—BOC-amino-4 méthyl-6 heptanoate de méthyle formé et on le lave avec de l'hexane. On reprend le précipité mouillé avec un mélange d'eau/hexane et on ajoute de l'acide chlorhydrique 1M jusqu'à pH = 2 à 3.

On extrait 2 fois à l'hexane et on lave la phase organique avec une solution de bicarbonate de sodium à 10 %. Après séchage et évaporation, on isole 86,2 g de (4S)-oxo-3 N—BOC-amino-4 méthyl-6 heptanoate de méthyle sous forme huileuse.

Rendement : 62 % calculé au départ de la N—BOC—L-leucine.
$\alpha_D^{25}$ = —55,1° (C = 1, méthanol)
R.M.N. et C.C.M. : conformes.

De manière analogue à celle décrite précédemment, on a préparé les composés suivants, les rendements étant calculés à partir du N—BOC acide aminé L correspondant :

(4S)-oxo-3 N—BOC-amino-4 phényl-5 pentanoate de méthyle
Rendement : 51 %
P.F. : 85-86 °C (hexane)
$\alpha_D^{25}$ = —60,4° (C = 1, méthanol)

(4S)-oxo-3 N—BOC-amino-4 cyclohexyl-5 pentanoate de méthyle
Rendement : 76 %
Huileux
$\alpha_D^{25}$ = — 34,3° (C = 1, méthanol)

(4S)-oxo-3 N—BOC-amino-4 phényl-4 butyrate de méthyle
Rendement : 52 %
P.F. : 97-98 °C (toluène)
$\alpha_D^{25}$ = + 77° (C = 1, méthanol)

(4S)-oxo-3 N-BOC-amino-4 méthyl-5 hexanoate de méthyle
Rendement : 75 %
Huileux
$\alpha_D^{25}$ = — 43,4° (C = 1, méthanol)

(4S)-oxo-3 N—BOC-amino-4 octanoate de méthyle
Rendement : 59 %
P.F. : 47-48 °C (n-pentane)
$\alpha_D^{25}$ = — 45,7° (C = 1, méthanol)

(4S)-oxo-3 N—BOC-amino-4 pentanoate de méthyle
Rendement : 75 %
$\alpha_D^{21}$ = — 52,6° (C = 1, méthanol)

(4S(—OXO—3 N—BOC-amino-4 (indolyl-3)-5 pentanoate de méthyle
Rendement : 74 %
$\alpha_D^{21}$ = — 34,1° (C = 1, méthanol)

Exemple II

Préparation du (4S)-oxo-3 N—BOC-amino-4 méthyl-6 heptanoate de méthyle

A la suspension d'énolate magnésien du malonate acide de méthyle obtenue au paragraphe D précédent (préparée à partir de 23,5 g de malonate acide de méthyle), on ajoute, à température ambiante et sous agitation, la solution d'imidazolide obtenue au paragraphe B précédent (préparée à partir de 11,5 g de N—BOC—L-leucine).

On obtient une suspension dans laquelle on verse, sous agitation, 300 ml de diméthylsulfoxyde anhydre et on observe une dissolution complète.

Après 4 heures d'agitation à température ambiante, on hydrolyse avec de l'acide chlorhydrique 1M à température de 20 °C (pH = 5 à 6). On agite 30 minutes afin de parfaire l'hydrolyse. On décante et extrait 3 fois à l'hexane. On lave la phase organique successivement avec de l'eau, de l'eau bicarbonatée puis de l'eau. Après séchage sur sulfate de sodium, on évapore à sec pour obtenir 26 g de (4S)-oxo-3 N—BOC-amino-4 méthyl-6 heptanoate de méthyle brut sous forme huileuse (rendement : 90 %). On reprend ce dérivé d'heptanoate de méthyle brut avec 100 ml d'éther isopropylique, 200 ml d'hexane et 100 ml d'eau. A température inférieure à 20 °C, on ajoute, sous agitation, 50 ml d'une solution d'hydroxyde de sodium à 30 %. Après 15 minutes d'agitation, on filtre le précipité de sel sodique de (4S)-oxo-3 N—BOC-amino-4 méthyl-6 heptanoate de méthyle et on lave avec de l'hexane.

On reprend le précipité mouillé avec un mélange eau/hexane et on ajoute de l'acide chlorhydrique 1M jusqu'à pH = 2 à 3. On extrait 2 fois avec de l'hexane et on lave la phase organique avec une solution de bicarbonate de sodium à 10 %. Après séchage et évaporation, on isole 18 g de (4S)-oxo-3 N—BOC-amino-4 méthyl-6 heptanoate de méthyle sous forme huileuse, qui se solidifie au repos.

Rendement : 63 % calculé au départ de la N—BOC—L-leucine.

P.F. : 32-34 °C (pentane à environ —30 °C)

$\alpha_D^{25}$ = —55,1° (C = 1, méthanol)

R.M.N. et C.C.M. : conformes

En utilisant le même procédé que ci-dessus mais en remplaçant le diméthyl-sulfoxyde par le N,N-diméthylformamide, on obtient le (4S)-oxo-3 N—BOC-amino-4 méthyl-6 heptanoate de méthyle avec un rendement de 53 %.

Exemple III

Préparation du (4S)-oxo-3 N—BOC-amino-4 méthyl-6 heptanoate de méthyle

On dissout dans de l'eau, les 9,5 g de sel sodique de (4S)-oxo-3 N—BOC-amino-4 méthyl-6 heptanoate de méthyle obtenus à l'Exemple 1 et on ajoute de l'acide chlorhydrique 1M jusqu'à pH = 2 à 3. On extrait 2 fois avec de l'hexane et on lave la phase organique avec une solution de bicarbonate de sodium à 10 %. Après séchage et évaporation, on isole 8,5 g de (4S)-oxo-3 N—NBOC-amino-4 méthyl-6 heptanoate de méthyle pur.

Rendement : 96 % calculé à partir du sel sodique ou 60 % calculé à partir de la N—BOC—L-leucine.

De la même manière, mais au départ des 56,5 g de sel sodique de (4S)-oxo-3 N—BOC-amino-4 méthyl-6 heptanoate d'éthyle obtenus à l'Exemple 1, on obtient 47,4 g de (4S)-oxo-3 N—BOC-amino-4 méthyl-6 heptanoate d'éthyle.

Rendement : 90 % calculé à partir du sel sodique ou 56 % calculé à partir de la N—BOC—L-leucine.

Exemple IV

Préparation du (3S,4S)-hydroxy-3 N—BOC-amino-4 méthyl-6 heptanoate de méthyle

On dissout dans 400 ml de méthanol anhydre les 86,2 g de (4S)-oxo-3 N—BOC-amino-4 méthyl-6 heptanoate de méthyle pur obtenu à partir de son sel sodique comme décrit à l'Exemple I. Après addition d'environ 6 g de nickel de Raney, on hydrogène durant 72 heures sous une pression d'environ 7 kg et à température d'environ 20 °C.

Après cette opération, on filtre et on évapore à sec pour obtenir 81,7 g d'un mélange de deux diastéréoisomères (3S,4S ; 3R,4S) d'hydroxy-3 N—BOC-amino-4 méthyl-6 heptanoate de méthyle sous forme huileuse (rendement : 94 % au départ du dérivé oxo-3 ou 58,2 % au départ de la N—BOC—L-leucine).

On sépare alors cette huile par chromatographie sur colonne de gel de silice (diamètre : 90 mm, hauteur : 500 mm) en utilisant un mélange acétate d'éthyle/hexane 10 : 90.

Après évaporation des différentes fractions, on isole les deux diastéréoisomères qui cristallisent au repos.

On obtient ainsi :

a) 30,8 g d'isomère (3S, 4S).

Rendement : 37 % calculé au départ du dérivé oxo-3

P.F. : 59 °C

$\alpha_D^{25}$ = —39,5° (C = 1, méthanol)

R.M.N. : conforme

b) 48,1 g d'isomère (3R,4S)

Rendement : 58,5 % calculé au départ du dérivé oxo-3

P.F. : 63,5 °C

$\alpha_D^{25}$ = —23,5° (C = 1, méthanol)

R.M.N. : conforme.

Exemple V

Préparation du (3S,4S)-hydroxy-3 N—BOC-amino-4 méthyl-6 heptanoate de méthyle

On dissout 6 g de sel sodique de (4S)-oxo-3 N—BOC-amino-4 méthjyl-6 heptanoate de méthyle dans 150 ml de méthanol. Après addition de 1 g de nickel de Raney, on hydrogène sous une pression de 7 kg durant 24 heures.

On neutralise l'alcalinité par de l'acide acétique et on filtre. Après évaporation, on reprend par l'hexane afin d'éliminer les sels minéraux puis on évapore l'hexane.

On obtient ainsi 5,3 g de mélange de deux diastéréoisomères (3S,4S ; 3R,4S) d'hydroxy-3 N—BOC-amino-4 méthyl-6 heptanoate de méthyle brut (rendement : 91 % au départ du sel sodique ou 56,4 % au départ de la N—BOPC—L-leucine).

On sépare alors ce mélange brut par chromatographie sur colonne de silice (diamètre : 90 mm, hauteur : 500 mm) en utilisant un mélange acétate d'éthyle/hexane 10 : 90.

Après évaporation des différentes fractions, on isole les deux diastéréoisomères qui cristallisent au repos.

On obtient ainsi :

a) 1,73 g d'isomères (3S,4S)
Rendement : 30 % calculé au départ du sel sodique
P.F. : 59 °C
$\alpha_D^{25}$ = — 36,3° (C = 1, méthanol)
C.C:M: : conforme

b) 3,2 g d'isomère (3R, 4S)
Rendement : 55 %
P.F. : 63 °C
$\alpha_D^{25}$ = — 21,85° (C = 1, méthanol)

De la même manière que celle décrite précédemment, on a préparé les composés suivants, les rendements étant calculés à partir du dérivé (4S)-oxo-3 N—BOC-amino-4 correspondant.

(3S,4S)-hydroxy-3 N—BOC-amino-4 phényl-5 pentanoate de méthyle

Rendement : 20 %
P.F. : 96-97 °C (n-pentane)
$\alpha_D^{25}$ = —34,9° (C = 1, méthanol)

(3R,4S)-hydroxy-3 N—BBOC-amino-4 phényl-5 pentanoate de méthyle

Rendement : 30 %
P.F. : 130-131 °C (n-pentane)
$\alpha_D^{25}$ = — 16,9° (C = 1, méthanol)

(3S,4S)-hydroxy-3 N—BOC-amino-4 cyclohexyl-5 pentanoate de méthyle

Rendement : 15 %
P.F. : 65-66 °C (n-pentane)
$\alpha_D^{25}$ = — 32,8° (C = 1, méthanol)

(3R,4S)-hydroxy-3 N—BOC-amino-4 cyclohexyl-5 pentanoate de méthyle

Rendement : 22 %
P.F. : 81-82 °C (n-pentane)
$\alpha_D^{25}$ = — 22,2° (C = 1, méthanol)

(3S,4S)-hydroxy-3 N—BOC-amino-4 phényl-4 butyrate de méthyle

Rendement : 12 %
P.F. : 94-95 °C (éther diisopropylique)
$\alpha_D^{25}$ = + 7,9° (C = 1, méthanol)

(3R,4S)-hydroxy-3 N—BOC-amino-4 phényl-4 butyrate de méthyle

Rendement : 45 %
P.F. : 94-95 °C (éther diisopropylique)
$\alpha_D^{25} = + 1{,}8°$ (C = 1, méthanol)

(3S,4S)-hydroxy-3 N—BOC-amino-4 méthyl-5 hexanoate de méthyle

Rendement : 22 %
P.F. : 58-59 °C (n-pentane)
$\alpha_D^{25} = - 43{,}85°$ (C = 1, méthanol)

(3R,4S)-hydroxy-3 N—BOC-amino-4 méthyl-5 hexanoate de méthyle

Rendement : 44 %
P.F. : 64-65 °C (n-pentane)
$\alpha_D^{25} = + 11{,}25°$ (C = 1, méthanol)

(3S,4S)-hydroxy-3 N—BOC-amino-4 octanoate de méthyle

Rendement : 35 %
P.F. : 54-55 °C (n-pentane)
$\alpha_D^{25} = - 15{,}4°$ (C = 1, méthanol)

(3R,4S)-hydroxy-3 N—BOC-amino-4 octanoate de méthyle

Rendement : 38 %
P.F. : 78-79 °C (n-pentane)
$\alpha_D^{25} = - 15{,}4°$ (C = 1, méthanol)

Exemple VI

Préparation du (3S,4S)-hydroxy-3 N—BOC-amino-4 méthyl-6 heptanoate d'éthyle

On dissout dans 80 ml de méthanol anhydre 8,3 g de (4S)-oxo-3 N—BOC-amino-4 méthyl-6 heptanoate d'éthyle pur obtenu à partir de son sel sodique comme décrit l'Exemple II. Après addition d'environ 1 g de nickel de Raney, on hydrogène durant 48 heures sous une pression d'environ 7 kg.

Après cette opération, on filtre et on évapore à sec pour obtenir un mélange de deux diastéréoisomères (3S,4S ; 3R,4S) d'hydroxy-3 N—BOC-amino-4 méthyl-6 heptanoate d'éthyle avec un rendement de 90 %.

On sépare cette huile par chromatographie sur colonne de gel de silice (diamètre : 90 mm, hauteur : 500 mm) en utilisant un mélange acétate d'éthyle/hexane 10 : 90.

Après évaporation des différentes fractions, on isole les deux diastéréoisomères sous forme huileuse.

On obtient ainsi :

a) 3,3 g d'isomère (3S,4S)

Rendement : 40 % calculé au départ du (4S)-oxo-3 N—BOC-amino-4 méthyl-6 heptanoate d'éthyle
$\alpha_D^{25} = - 31{,}7°$ (C = 1, méthanol)
R.M.N. et C.C.M. : conformes

b) 4,1 g d'isomère (3R,4S)

Rendement : 50 % calculé au départ du (4S)-oxo-3 N—BOC-amino-4 méthyl-6 heptanoate d'éthyle
$\alpha_D^{25} = - 14{,}7°$ (C = 1, méthanol)
R.M.N. et C.C.M. : conformes

**Revendications**

1. Dérivés d'acide amino-4 butanoïque de formule générale :

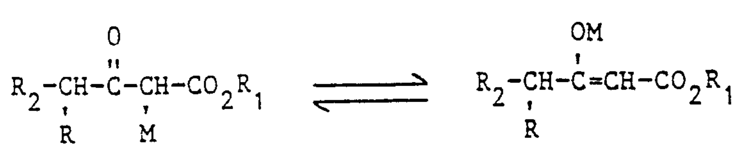

dans laquelle :

M représente un atome de métal alcalin,

R représente un groupement amino protégé,

$R_1$ représente un groupement labile,

$R_2$ représente hydrogène, un radical alkyle, linéaire ou ramifié, ayant de 1 à 6 atomes de carbone, un radical méthyloxyalkyle inférieur, un radical méthylthioalkyle inférieur, un radical (alkyle inférieur) aminoalkyle inférieur un radical di-(alkyle inférieur) aminoalkyle inférieur, un radical hydroxyalkyle inférieur ou $R_2$ représente l'un des radicaux de formules générales :

$$Cy—A,\ Cy—O—A'—,\ R—A'—\ ou\ R_3S—A'—$$

dans laquelle :

Cy représente un radical hydrocarboné aromatique ou alicyclique ou un radical hétérocyclique contenant un atome d'oxygène ou de soufre ou un ou deux atomes d'azote, Cy étant éventuellement mono-, di- ou tri-substitué par des radicaux hydroxy, alkyle inférieur, alkoxy inférieur, trifluorométhyle, nitro ou halogéno,

A représente une liaison simple ou un radical alkylène, linéaire ou ramifié, ayant de 1 à 5 atomes de carbone,

A' représente un radical alkylène, linéaire ou ramifié, ayant de 1 à 5 atomes de carbone,

$R_3$ représente un groupement S-protecteur.

2. Dérivés d'acide amino-4 butanoïque selon la Revendication 1 dans laquelle $R_2$ représente un radical méthyle, éthyle, n-propyle, isopropyle, n-butyle, isobutyle, tertiobutyle, méthyl-1 propyle, n-pentyle ou n-hexyle, méthoxyéthyle, méthylthioéthyle, méthylaminoéthyle, éthylaminoéthyle, isopropylaminoéthyle, tertiobutylaminoéthyle, diméthylaminoéthyle, diéthylaminoéthyle, di-n-propylaminoéthyle, di-n-butylaminoéthyle, hydroxyméthyle, hydroxy-1 éthyle, phényle, hydroxyphényle, méthylphényle, monofluoro-, monochloro- ou mono-bromo-phényle, di-fluoro-, di-chloro- ou di-bromo-phényle, monométhoxy, di-méthoxy ou tri-méthoxy-phényle, trifluorométhylphényle, nitrophényle, benzyle, phénétyle, cyclohexyle, cyclohexylméthyle, picolyle, pipéridinométhyle, indolylméthyle, 1H-imidazolylméthyle, phénoxyéthyle, phénoxy-n-propyle, amino-4 butyle N-protégé ou mercaptométhyle S-protégé.

3. Dérivés d'acide amino-4 butanoïque selon la Revendication 1 ou 2 dans laquelle $R_2$ représente méthyle.

4. Dérivés d'acide amino-4 butanoïque selon la Revendication 1 ou 2 dans laquelle $R_2$ représente éthyle.

5. Dérivés d'acide amino-4 butanoïque selon la Revendication 1 ou 2 dans laquelle $R_2$ représente n-propyle.

6. Dérivés d'acide amino-4 butanoïque selon la Revendication 1 ou 2 dans laquelle $R_2$ représente isopropyle.

7. Dérivés d'acide amino-4 butanoïque selon la Revendication 1 ou 2 dans laquelle $R_2$ représente n-butyle.

8. Dérivés d'acide amino-4 butanoïque selon la Revendication 1 ou 2 dans laquelle $R_2$ représente isobutyle.

9. Dérivés d'acide amino-4 butanoïque selon la Revendication 1 ou 2 dans laquelle $R_2$ représente méthyl-1 propyle.

10. Dérivés d'acide amino-4 butanoïque selon la Revendication 1 ou 2 dans laquelle $R_2$ représente phényle.

11. Dérivés d'acide amino-4 butanoïque selon la Revendication 1 ou 2 dans laquelle $R_2$ représente benzyle.

12. Dérivés d'acide amino-4 butanoïque selon la Revendication 1 ou 2 dans laquelle $R_2$ représente hydroxy-4 phényle.

13. Dérivés d'acide amino-4 butanoïque selon la Revendication 1 ou 2 dans laquelle $R_2$ représente cyclohexyle.

14. Dérivés d'acide amino-4 butanoïque selon la Revendication 1 ou 2 dans laquelle $R_2$ représente cyclohexylméthyle.

15. Dérivés d'acide amino-4 butanoïque selon la Revendication 1 ou 2 dans laquelle $R_2$ représente indolyl-3 méthyle.

16. Dérivés d'acide amino-4 butanoïque selon la Revendication 1 ou 2 dans laquelle $R_2$ représente 1H-imidazolyl-4 méthyle.

17. Dérivés d'acide amino-4 butanoïque selon l'une des Revendications 1 à 16 dans laquelle R représente le groupement tertiobutyloxycarbonylamino.

18. Dérivés d'acide amino-4 butanoïque selon l'une des Revendications 1 à 16 dans laquelle $R_1$ représente méthyle, éthyle, n-propyle, isopropyle, n-butyle, isobutyle, tertiobutyle ou xylyle.

19. Dérivés d'acide amino-4 butanoïque selon l'une des Revendications 1 à 16 dans laquelle M représente sodium.

20. Procédé de préparation de dérivés d'acide amino-4 butanoïque selon l'une des Revendications 1

à 19 caractérisé en ce que l'on fait réagir en milieu aqueux et à température inférieure à 20 °C, un ester de dérivé d'acide (4S)-oxo-3 amino-4 butanoïque N-protégé de formule générale :

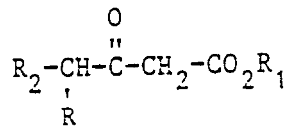

dans laquelle R, $R_1$ et $R_2$ ont la même signification que précédemment, avec un hydroxyde de métal alcalin, puis on sépare le sel formé du milieu réactionnel.

21. Procédé selon la Revendication 20 caractérisé en ce que l'on obtient l'ester de dérivé d'acide (4S)-oxo-3 amino-4 butanoïque N-protégé par réaction entre un imidazolide N-protégé de formule générale :

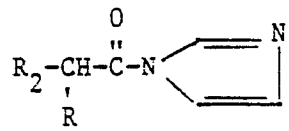

dans laquelle R et $R_2$ ont la même signification que dans la Revendication 20, avec un énolate magnésien d'un monoester d'acide malonique de formule générale :

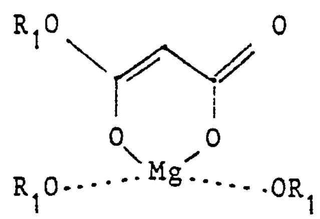

dans laquelle $R_1$ a la même signification que dans la Revendication 20, la réaction ayant lieu à température ambiante et dans un éther éventuellement en présence d'un solvant aprotique, ce qui donne un complexe que l'on hydrolyse en présence d'un acide fort pour fournir le composé dérivé.

22. Application des dérivés d'acide amino-4 butanoïque selon l'une des Revendications 1 à 19 à la purification des esters de dérivés d'acide (4S)-oxo-3 amino-4 butanoïque N-protégés correspondants obtenus à l'état brut caractérisée en ce que l'on forme, au moyen d'un hydroxyde de métal alcalin, le sel de métal alcalin des esters de dérivés d'acide (4S)-oxo-3 amino-4 butanoïque N-protégés on isole le sel formé puis on régénère les esters de dérivés d'acide (4S)-oxo-3 amino-4 butanoïque N-protégés par acidification au moyen d'un acide fort.

23. Application des dérivés d'acide amino-4 butanoïque selon l'une des Revendications 1 à 19 à la préparation des diastéréosiomères (3S,4S) et (3R,4S) des esters de dérivés d'acide hydroxy-3 amino-4 butanoïque N-protégés caractérisée en ce que l'on réduit lesdits dérivés d'acide amino-4 butanoïque pour obtenir les esters de dérivés d'acide (3S,4S ; 3R,4S)-hydroxy-3 amino-4 butanoïque N-protégés sous forme de diastéréoisomères que l'on sépare ultérieurement.

24. Dérivés d'acide (4S)-amino-4 butanoïque de formule générale :

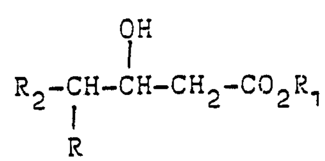

dans laquelle :

R représente un groupement amino protégé

$R_1$ représente un groupement labile

$R_2$ représente un groupe phényle ou cyclohexyle éventuellement substitué par un groupe hydroxy, alkyle ayant de 1 à 4 atomes de carbone ou alcoxy ayant de 1 à 4 atomes de carbone.

25. Dérivés d'acide amino-4 butanoïque selon la revendication 24, dans laquelle $R_2$ représente un radical phényle.

26. Dérivés d'acide amino-4 butanoïque selon la revendication 24, dans laquelle $R_2$ représente un radical hydroxy-4 phényle.

27. Dérivés d'acide amino-4 butanoïque selon la revendication 24, dans laquelle $R_2$ représente cyclohexyle.

28. Dérivés d'acide amino-4 butanoïque selon la revendication 24, dans laquelle R représente un radical tertiobutoxycarbonyle.

29. Dérivés d'acide amino-4 butanoïque selon la revendication 24, dans laquelle $R_1$ représente le groupement méthyle.

30. (3S,4S)-hydroxy-3N-BOC-amino-4 phényl-4 butanoate de méthyle.

31. (3R,4S)-hydroxy-3N-BOC-amino-4 phényl-4 butanoate de méthyle.

## Claims

1. Derivatives of 4-aminobutanoic acid of general formula :

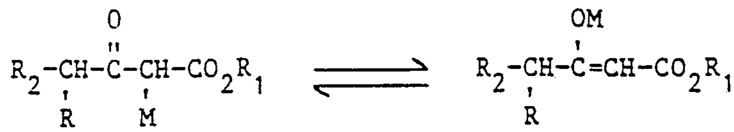

wherein

M represents an alkali metal atom,

R represents a protected amino group,

$R_1$ represents a labile group,

$R_2$ represents hydrogen, an alkyl radical, straightchained or branched, having 1 to 6 carbon atoms, a methoxy lower alkyl radical, a methylthio lower alkyl radical, a (lower alkyl) amino lower alkyl radical, a di-(lower alkyl) amino lower alkyl radical, a hydroxy lower alkyl radical or $R_2$ represents one of the radicals of general formula :

$$Cy—A,\ Cy—O—A'—,\ R—A'—\ \text{or}\ R_3S—A'—$$

wherein

Cy represents an aromatic or alicyclic hydrocarbon radical or a heterocyclic radical containing an oxygen or sulphur atom or one or two nitrogen atoms, Cy being optionally mono-, di- or trisubstituted by hydroxy, lower alkyl, lower alkoxy, trifluoromethyl, nitro or halide radicals,

A represents a single bond or a straightchained or branched alkylene radical, having 1 to 5 carbon atoms,

A' represents a straight-chained or branched alkylene radical, having 1 to 5 carbon atoms,

$R_3$ represents an S-protective group.

2. Derivatives of 4-aminobutanoic acid according to claim 1 wherein $R_2$ represents a methyl, ethyl, n-propyl, isopropyl, n-butyl, isobutyl, tert.butyl, 1-methyl propyl, n-pentyl or n-hexyl, methoxyethyl, methylthioethyl, methylaminoethyl, ethylaminoethyl, isopropylaminoethyl, tert.butylaminoethyl, dimethylaminoethyl, diethylaminoethyl, di-n-propylaminoethyl, di-n-butylaminoethyl, hydroxymethyl, 1-hydroxy-ethyl, phenyl, hydroxyphenyl, methylphenyl, mono-fluoro-, mono-chloro- or monobromophenyl, di-fluoro-, di-chloro- or di-bromo-phenyl, mono-methoxy-, di-methoxy- or tri-methoxyphenyl, trifluoromethylphenyl, nitrophenyl, benzyl, phenethyl, cyclohexyl, cyclohexylmethyl, picolyl, piperidinomethyl, indolylmethyl, 1H-imidazolylmethyl, phenoxyethyl, phenoxy-n-propyl, N-protected 4-aminobutyl or S-protected mercaptomethyl radical.

3. Derivatives of 4-aminobutanoic acid according to claim 1 or 2 wherein $R_2$ represents methyl.

4. Derivatives of 4-aminobutanoic acid according to claim 1 or 2 wherein $R_2$ represents ethyl.

5. Derivatives of 4-aminobutanoic acid according to claim 1 or 2 wherein $R_2$ represents n-propyl.

6. Derivatives of 4-aminobutanoic acid according to claim 1 or 2 wherein $R_2$ represents isopropyl.

7. Derivatives of 4-aminobutanoic acid according to claim 1 or 2 wherein $R_2$ represents n-butyl.

8. Derivatives of 4-aminobutanoic acid according to claim 1 or 2 wherein $R_2$ represents isobutyl.

9. Derivatives of 4-aminobutanoic acid according to claim 1 or 2 wherein $R_2$ represents 1-methylpropyl.

10. Derivatives of 4-aminobutanoic acid according to claim 1 or 2 wherein $R_2$ represents phenyl.

11. Derivatives of 4-aminobutanoic acid according to claim 1 or 2 wherein $R_2$ represents benzyl.

12. Derivatives of 4-aminobutanoic acid according to claim 1 or 2 wherein $R_2$ represents 4-hydroxyphenyl.

13. Derivatives of 4-aminobutanoic acid according to claim 1 or 2 wherein $R_2$ represents cyclohexyl.

14. Derivatives of 4-aminobutanoic acid according to claim 1 or 2 wherein $R_2$ represents cyclohexylmethyl.

15. Derivatives of 4-aminobutanoic acid according to claim 1 or 2 wherein $R_2$ represents 3-indolylmethyl.

16. Derivatives of 4-aminobutanoic acid according to claim 1 or 2 wherein $R_2$ represents 1H-4-imidazolylmethyl.

17. Derivatives of 4-aminobutanoic acid according to any one of claims 1 to 16 wherein R represents the tert.butyloxycarbonylamino group.

18. Derivatives of 4-aminobutanoic acid according to any one of claims 1 to 16 wherein $R_1$ represents methyl, ethyl, n-propyl, isopropyl, n-butyl, isobutyl, tert.butyl or xylyl.

19. Derivatives of 4-aminobutanoic acid according to any one of claims 1 to 16 wherein M represents sodium.

20. Process for preparing derivatives of 4-aminobutanoic acid according to any one of claims 1 to 19, characterised in that an N-protected ester derivative of (4S)-3-oxo-4-amino-butanoic acid of general formula

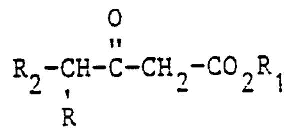

wherein R, $R_1$ and $R_2$ are as hereinbefore defined, is reacted in an aqueous medium and at a temperature below 20 °C with an alkali metal hydroxide, and the salt formed from the reaction medium is then separated.

21. Process according to claim 20, characterised in that the N-protected ester derivative of (4S)-3-oxo-4-aminobutanoic acid is obtained by reaction between an N-protected imidazolide of general formula

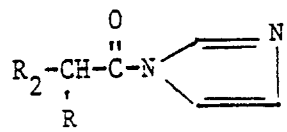

wherein R and $R_2$ have the same meaning as in claim 20, and a magnesium enolate of a monoester of malonic acid of general formula

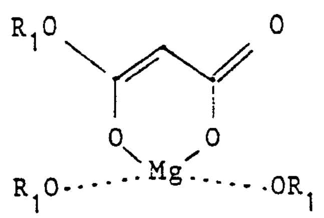

wherein $R_1$ have the same meaning as in claim 20, the reaction taking place at ambient temperature and in an ether, optionally in the presence of an aprotic solvent, resulting in a complex which is hydrolysed in the presence of a strong acid in order to obtain the desired compound.

22. Use of the derivatives of 4-aminobutanoic acid according to any one of claims 1 to 19 for the purification of N-protected ester derivatives of (4S)-3-oxo-4-aminobutanoic acid, obtained from the crude state, characterised in that, by means of an alkali metal hydroxide, the alkali metal salt of N-protected ester derivatives of (4S)-3-oxo-4-aminobutanoic acid is formed ; the salt thus formed is isolated and then the N-protected ester derivatives of (4S)-3-oxo-4-amino butanoic acid are regenerated by acidification with a strong acid.

23. Use of derivatives of 4-aminobutanoic acid according to any one of claims 1 to 19 for the preparation of diastereoisomers (3S,4S) and (3R,4S) of N-protected ester derivatives of 3-hydroxy-4-aminobutanoic acid, characterised in that said derivatives of 4-aminobutanoic acid are reduced in order to obtain the N-protected ester derivative of (3S,4S ; 3R,4S)-3-hydroxy-4-aminobutanoic acid in the form of diastereoisomers which are later separated;

24. Derivatives of (4S)-4-aminobutanoic acid of general formula

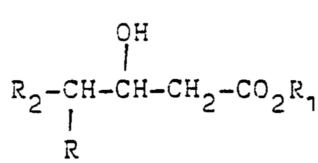

wherein

R represents a protected amino group

$R_1$ represents a labile group

$R_2$ represents a phenyl or cyclohexyl group, optionally substituted by a hydroxy or alkyl group having 1 to 4 carbon atoms or an alkoxy group having 1 to 4 carbon atoms.

25. Derivatives of 4-aminobutanoic acid according to claim 24, wherein $R_2$ represents a phenyl radical.

26. Derivative of 4-aminobutanoic acid according to claim 24, wherein $R_2$ represents a 4-hydroxyphenyl radical.

27. Derivative of 4-aminobutanoic acid according to claim 24, wherein $R_2$ represents cyclohexyl.

28. Derivative of 4-aminobutanoic acid according to claim 24, wherein R represents a tert.butoxycarbonyl radical.

29. Derivative of 4-aminobutanoic acid according to claim 24, wherein $R_1$ represents the methyl group.

30. Methyl (3S,4S)-3N-hydroxy-BOC-4-amino-4-phenylbutanoate.

31. Methyl (3R,4S)-3N-hydroxy-BOC-4-amino-4-phenylbutanoate.

## Patentansprüche

1. 4-Aminobutansäure-Derivate der allgemeinen Formel :

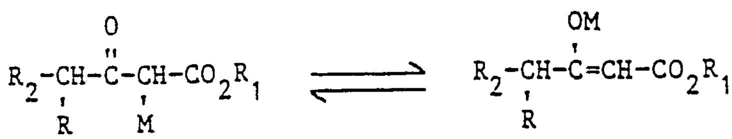

in der bedeuten :

M ein Alkalimetallatom,

R eine geschützte Aminogruppe,

$R_1$ eine labile Gruppe,

$R_2$ Wasserstoff, geradkettiges oder verzweigtes Alkyl mit 1 bis 6 Kohlenstoffatomen, niedriges Methyloxyalkyl, niedriges Methylthioalkyl, niedriges (niedriges Alkyl)-aminoalkyl, niedriges Di-(niedriges Alkyl)-aminoalkyl, niedriges Hydroxyalkyl oder einen der Reste der folgenden allgemeinen Formeln :

$$Cy—A,\ Cy—O—A'—,\ R—A'—\ \text{oder}\ R_3S—A'—$$

in denen bedeuten :

Cy einen aromatischen oder alicyclischen Kohlenwasserstoffrest oder einen heterocyclischen Rest mit einem Sauerstoff- oder Schwefelatom oder einem oder zwei Stickstoffatomen die ggfs. mit einem, zwei oder drei Hydroxyl-, niedrigen Alkyl-, niedrigen Alkoxy-, Trifluormethyl- oder Nitrogruppen oder Halogenatomen substituiert sein können,

A eine Einfachbindung oder einen geradkettigen oder verzweigten Alkylenrest mit 1 bis 5 Kohlenstoffatomen,

A' einen geradkettigen oder verzweigten Alkylenrest mit 1 bis 5 Kohlenstoffatomen und

$R_3$ eine S-Schutzgruppe.

2. 4-Aminobutansäure-Derivate nach Anspruch 1, in denen $R_2$ Methyl, Ethyl, n-Propyl, Isopropyl, n-Butyl, Isobutyl, t-Butyl, 1-Methylpropyl, n-Pentyl oder n-Hexyl, Methoxyethyl, Methylthioethyl, Methylaminoethyl, Ethylaminoethyl, Isopropylaminoethyl, t-Butylaminoethyl, Dimethylaminoethyl, Diethylaminoethyl, Di-n-propyl-(aminoethyl), Di-(n-butyl)-aminoethyl, Hydroxymethyl, 1-Hydroxyethyl, Phenyl, Hydroxyphenyl, Methylphenyl, Monofluor-, Monochlor- oder Monobromphenyl, Difluor-, Dichlor- oder Dibromphenyl, Monomethoxy-, Dimethoxy- oder Trimethoxyphenyl, Trifluormethylphenyl, Nitrophenyl, Benzyl, Phenethyl, Cyclohexyl, Cyclohexylmethyl, Picolyl, Piperidinomethyl, Indolylmethyl, 1H-Imidazolylmethyl, Phenoxyethyl, Phenoxy-n-propyl, N-geschütztes 4-Aminobutyl oder S-geschütztes Mercaptomethyl bedeutet.

3. 4-Aminobutansäure-Derivate nach Anspruch 1 oder 2, in denen $R_2$ Methyl ist.

4. 4-Aminobutansäure-Derivate nach Anspruch 1 oder 2, in denen $R_2$ Ethyl ist.

5. 4-Aminobutansäure-Derivate nach Anspruch 1 oder 2, in denen $R_2$ n-Propyl ist.

6. 4-Aminobutansäure-Derivate nach Anspruch 1 oder 2, in denen $R_2$ Isopropyl ist.

7. 4-Aminobutansäure-Derivate nach Anspruch 1 oder 2, in denen $R_2$ n-Butyl ist.

8. 4-Aminobutansäure-Derivate nach Anspruch 1 oder 2, in denen $R_2$ Isobutyl ist.

9. 4-Aminobutansäure-Derivate nach Anspruch 1 oder 2, in denen $R_2$ 1-Methylpropyl ist.

10. 4-Aminobutansäure-Derivate nach Anspruch 1 oder 2, denen $R_2$ Phenyl ist.

11. 4-Aminobutansäure-Derivate nach Anspruch 1 oder 2, in denen $R_2$ Benzyl ist.

12. 4-Aminobutansäure-Derivate nach Anspruch 1 oder 2, in denen $R_2$ 4-hydroxyphenyl ist.

13. 4-Aminobutansäure-Derivate nach Anspruch 1 oder 2, in denen $R_2$ Cyclohexyl ist.

14. 4-Aminobutansäure-Derivate nach Anspruch 1 oder 2, in denen $R_2$ Cyclohexylmethyl ist.

15. 4-Aminobutansäure-Derivate nach Anspruch 1 oder 2, in denen $R_2$ 3-Indolylmethyl ist.

16. 4-Aminobutansäure-Derivate nach Anspruch 1 oder 2, in denen $R_2$ 1H-4-Imidazolylmethyl ist.

17. 4-Aminobutansäure-Derivate nach einem der Ansprüche 1 bis 16, in denen R t-Butyloxycarbonylamino ist.

18. 4-Aminobutansäure-Derivate nach einem der Ansprüche 1 bis 16, in denen $R_1$ Methyl, Ethyl, n-Propyl, Isopropyl, n-Butyl, Isobutyl, t-Butyl oder Xylyl ist.

19. 4-Aminobutansäure-Derivate nach einem der Ansprüche 1 bis 16, in denen M Natrium ist.

20. Verfahren zur Herstellung der 4-Aminobutansäure-Derivate nach einem der Ansprüche 1 bis 19, gekennzeichnet durch Umsetzung in einem wäßrigen Medium und bei einer Temperatur unter 20 °C eines N-geschützten (4S)-3-Oxo-4-aminobutansäureesters der allgemeinen Formel

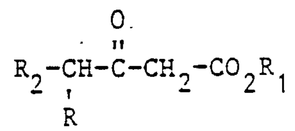

in der R, $R_1$ und $R_2$ die oben angegebene Bedeutung haben, mit einem Alkalimetallhydroxid und Abtrennung des gebildeten Salzes aus dem Reaktionsmedium.

21. Verfahren nach Anspruch 20, dadurch gekennzeichnet, daß der N-geschützte (4S)-3-Oxo-4-aminobutansäureester durch Umsetzung eines N-geschützten Imidazolids der allgemeinen Formel :

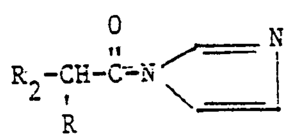

in der R und $R_2$ die in Anspruch 20 angegebene Bedeutung haben, mit einem Magnesiumenolat eines Malonsäure-monoesters der allgemeinen Formel :

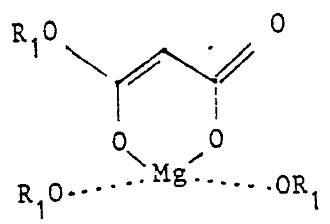

in der $R_1$ die in Anspruch 20 angegebene Bedeutung hat, erhalten wird, wobei die Reaktion bei Raumtemperatur und in einem Ether, ggfs. in Gegenwart eines aprotischen Lösungsmittels, zu einem Komplex durchgeführt wird, der in Gegenwart einer starken Säure zur gewünschten Verbindung hydrolisiert wird.

22. Verwendung der 4-Aminobutansäure-Derivate nach einem der Ansprüche 1 bis 19 zur Reinigung der entsprechenden, rohen, N-geschützten (4S)-3-Oxo-4-aminobutansäureester, gekennzeichnet durch Bildung des Alkalimetallsalzes der N-geschützten (4S)-3-Oxo-4-aminobutansäureester mit einem Alkalimetallhydroxid, Isolierung des gebildeten Salzes und Wiedergewinnung der N-geschützten (4S)-3-Oxo-4-aminobutansäureester durch Ansäuern mit einer starken Säure.

23. Verwendung der 4-Aminobutansäure-Derivate nach einem der Ansprüche 1 bis 19 zur Herstellung der (3S,4S)- und (3R,4S)-Diastereoisomeren der N-geschützten 3-Hydroxy-4-aminobutansäureester, gekennzeichnet durch Reduktion der 4-Aminobutansäure-Derivate zu den N-geschützten (3S,4S ; 3R,4S)-3-Hydroxy-4-aminobutansäureester in Form der Diastereoisomeren und folgende Trennung.

24. (4S)-4-Aminobutansäure-Derivate der allgemeinen Formel :

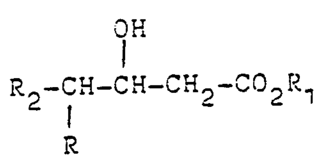

in der bedeuten :

R eine geschützte Aminogruppe,

$R_1$ eine labile Gruppe

und

$R_2$ ggfs. mit Hydroxyl, $C_{1-4}$-Alkyl oder $C_{1-4}$-Alkoxy substituiertes Phenyl oder Cyclohexyl.

25. 4-Aminobutansäure-Derivate nach Anspruch 24, in denen $R_2$ Phenyl ist.
26. 4-Aminobutansäure-Derivate nach Anspruch 24, in denen $R_2$ 4-Hydroxyphenyl ist.
27. 4-Aminobutansäure-Derivate nach Anspruch 24, in denen $R_2$ Cyclohexyl ist.
28. 4-Aminobutansäure-Derivate nach Anspruch 24, in denen R t-Butoxycarbonyl ist.
29. 4-Aminobutansäure-Derivate nach Anspruch 24, in denen $R_1$ Methyl ist.
30. (3S,4S)-3-Hydroxy-4-N-BOC-amino-4-phenylbutansäuremethylester.
31. (3R,4S)-3-Hydroxy-4-N-BOC-amino-4-phenylbutansäuremethylester.